Europäisches Patentamt

European Patent Office

Office européen des brevets

(19)

(11) Publication number : **0 434 347 A1**

(12) **EUROPEAN PATENT APPLICATION**

(21) Application number : 90313794.1

(22) Date of filing : 17.12.90

(51) Int. Cl.⁵ : **C07C 5/27, C07C 15/08**

(30) Priority : 18.12.89 US 452088

(43) Date of publication of application :
26.06.91 Bulletin 91/26

(84) Designated Contracting States :
BE DE FR GB IT NL

(71) Applicant : **MOBIL OIL CORPORATION**
3225 Gallows Road
Fairfax Virginia 22037 (US)

(72) Inventor : **Haag, Werner Otto**
**38 Pine Knoll Drive**
**Lawrenceville, New Jersey 08648 (US)**
Inventor : **Olson, David Harold**
**11 Morningside Drive**
**Pennington, New Jersey 08534 (US)**

(74) Representative : **Colmer, Stephen Gary et al**
**Mobil Court 3 Clements Inn**
**London WC2A 2EB (GB)**

(54) Xylene isomerization process.

(57)   The process relates to xylene isomerization, in the presence of ethylbenzene, catalyzed by a two component catalyst system, comprising two different zeolites, with selectivity for conversion based on the different pore structures of the two different zeolites.

EP 0 434 347 A1

# XYLENE ISOMERIZATION PROCESS

This invention relates to a xylene isomerization process.

Xylenes are valuable industrial chemicals derived primarily from aromatic naphthas such as petroleum reformates and pyrolysis gasolines. Petroleum reformates result from processing petroleum naphthas over a catalyst such as platinum on alumina at temperatures which favor dehydrogenation of naphthenes. Pyrolysis gasolines are liquid products resulting from steam cracking of hydrocarbons to manufacture ethylene, propylene, and the like.

Generally, regardless of the aromatic naphtha source, it has been the practice to subject the source to extraction with a solvent highly selective for aromatics to obtain an aromatic mixture of the benzene and alkylated benzenes present in the aromatic naphtha. The resulting extract can then be distilled to separate benzene, toluene and $C_8$ aromatics from higher boiling compounds in the extract. Benzene and toluene are recovered in high purity ; however, the $C_8$ fraction, containing valuable para-xylene, is a mixture of three xylene isomers with ethylbenzene. These mixtures will also contain $C_8$-$C_9$ paraffins, the amount of which is determined both by the source of the naphtha as well as the efficiency of the solvent extraction.

As commercial use of xylene has increased, there has been interest in isomerizing xylene to increase the yield of the desired ortho- and para-isomers. Para-xylene is of particular value as it is useful in the manufacture of terephthalic acid which is an intermediate in the manufacture of polyesters and synthetic fibers.

In practice, xylene isomerization is effected in conjunction with a xylene separation process. Thus, a virgin $C_8$ aromatics mixture is fed to such a combined process system, along with undesired isomers emerging from the product separation steps. The feed is charged to the isomerizing unit and the effluent isomerizate $C_8$ aromatics are sent to the product separation steps. The composition of isomerizer feed is then a function of the virgin $C_8$ aromatic feed, the product separation unit performance, and the isomerizer performance. The objective in the isomerization reactor is to bring the charge as near to the equilibrium concentration as may be feasible consistent with reaction times which do not give extensive cracking and disproportionation. The thermodynamic equilibrium varies slightly with temperature.

The degree of ethylbenzene conversion in a $C_8$ aromatic mixture is related to effective contact time. Hydrogen partial pressure can have a very significant effect on ethylbenzene conversion. Products formed from ethylbenzene include $C_8$ naphthenes, benzene from hydrocracking ethylbenzene and $C_9^+$ aromatics from disproportionation and from transalkylation with xylene, as well as total loss to other than $C_8$ molecular weight components, such as $C_5$ and lighter hydrocarbon byproducts.

The various xylene isomers exhibit different rates of isomerization and hence, with different feed composition situations, the rates of approach to equilibrium vary considerably. Loss of xylenes to other molecular weight products varies with contact time. By-products include toluene, $C_9$ and heavier aromatics and $C_5$ and lighter hydrocracking products.

Because of the deleterious effects of ethylbenzene build up in the loop manufacture of the xylenes and because of the great expense of removing it from mixed $C_8$ aromatics, a process which effects ethylbenzene conversion at a rate approaching that of xylene isomerization would be desirable provided xylene losses can be maintained at a reasonable level. Progress toward such a goal was provided by U.S. Patent No. 4,163,028 which describes a catalyst and its use in an isomerization process conducted at 427 to 540°C (800 to 1000°F). The catalyst described in U.S. Patent No. 4,163,028 comprises a zeolite having a constraint index of 1 to 12 and having a silica/alumina ratio of at least 500. An improved catalyst for that aforementioned goal was later described in U.S. Patent No. 4,312,790.

However, even the use of the catalysts of U.S. Patent Nos. 4,163,028 and 4,312,790 can result in undesirably high xylene losses, especially when xylene isomerization feeds containing greater than 15% ethylbenzene are processed under conditions which give greater than about 50% conversion of ethylbenzene per pass.

An object of the present invention is therefore to provide a process for selectively isomerizing xylenes while converting ethylbenzene to other products with little loss of xylenes via transalkylation.

Accordingly, the invention resides in a process for isomerizing a feed containing an aromatic $C_8$ mixture, which comprises xylene and from 0 to 50 percent ethylbenzene and in which the para-xylene is less than that at thermodynamic equilibrium, which process comprises contacting the feed with a catalyst system comprising component (A) and component (B) wherein :

component (A) comprises at least one zeolite and 0 to 10 weight percent of a hydrogenation metal, wherein said at least one zeolite produces less than 80 weight% equilibrium isomerization of para-xylene when tested under test conditions including a temperature of 370 to 480°C (700 to 900°F), a pressure of 100 to 4050 kPa (1 to 40 atmospheres), $H_2$ to hydrocarbon of 1 to 4, and a WHSV sufficient to produce 10 weight% ethylbenzene conversion with a feed of 14 weight% ethylbenzene, 10 weight% p-xylene, 53 weight% m-xylene and 23 weight% o-xylene ;

component (B) comprises a further zeolite, different from the zeolite of component (A) and 0 to 10 weight percent of a hydrogenation metal, wherein said further zeolite produces greater than 80 weight% equilibrium isomerization of para-xylene when tested under said test conditions ; and

the weight ratio of the zeolite of component (A) to the zeolite of component (B) ranges from 20/80 to 98/2 ;

said contacting being undertaken at a temperature of 200 to 600°C (400 to 1100°F), a pressure of 100 to 10450 kPa (0 to 1500 psig), a WHSV of 0.5 to 100 and a $H_2$/HC molar ratio of 0 to 10.

The catalyst system used in accordance with the invention has at least two components. One of the catalyst components (Component B) includes a zeolite which has a Constraint Index of 1-12 (described in US Patent No. 4016218) and which is highly selective and active for isomerization of the xylene components to thermodynamic equilibrium. The other component (Component A) of the catalyst system incudes a zeolite, different from the zeolite of Component (B), which also has a Constraint Index of 1-12 and which is highly selective for the conversion of ethylbenzene via dealkylation and disproportionation. The weight ratio of the catalyst component (A) to that of the catalyst component (B) ranges from 20 : 80 to 98 : 2.

More specifically, the zeolite of component (A) is characterized as producing less than 80 weight% of the equilibrium p-xylene concentration when tested under conditions including a temperature of 370 to 480°C (700 to 900°F), a pressure of 100 to 4050 kPa (1 to 40 atmospheres), $H_2$ to hydrocarbon ratio of 1 to 4 and a WHSV sufficient to give 10% ethylbenzene conversion with a feed consisting of 14 weight percent ethylbenzene, 10 weight percent p-xylene, 53 weight percent m-xylene and 23 weight percent o-xylene. Under the same test conditions when the zeolite B converts 10% of the ethylbenzene of the feed, it will produce greater than 80% of equilibrium p-xylene.

Each of the zeolites of the two components of the catalyst system will exhibit mutually exclusive xylene diffusional properties. Those properties can be identified by noting the time (in minutes) required to sorb 30% of the equilibrium capacity of ortho xylene at 120°C and at an o-xylene partial pressure of 4.5 ± 0.8 mm of mercury, a test described by Olson et al in U.S. Patent Nos. 4,117,026, 4,159,282 and Re. 31,782. Herein, that equilibrium capacity of ortho-xylene is defined as greater than 1 gram of xylene(s) per 100 grams of zeolite. The zeolite of component A preferably exhibits an o-xylene absorption time of greater than 50 and more preferably greater than 100 minutes while the zeolite of component (B) preferably exhibits an o-xylene sorption time of less than 50 minutes and more preferably less than 10 minutes.

Most preferably, the zeolite of component A is selected from ZSM-22, ZSM-23, ZSM-35 and ZSM-48, while the zeolite of component (B) is ZSM-5 or ZSM-11. ZSM-5 is described in detail in U.S. Pat. Nos. 3,702,886 and Re. 29,948. ZSM-11 is described in U.S. Pat. No. 3,709,979. ZSM-22 is described in U.S. Pat. Nos. 4,481,177 and 4,556,477. ZSM-23 is described in U.S. Pat. No. 4,076,842. ZSM-35 is described in U.S. Pat. No. 4,016,245. ZSM-48 is described in European Patent No. 15132.

The zeolite of each of the catalyst components (A) and (B) can be associated with a hydrogenation-dehydrogenation component such as nickel, palladium or platinum. The hydrogenation-dehydrogenation component is preferably a noble metal such as platinum, palladium, or another member of the platinum group such as rhodium, with platinum most preferred. Combinations of noble metals such as platinum-rhenium, platinum-palladium, platinum-iridium or platinum-iridium-rhenium together with combinations with non-noble metals, particularly of Groups VIA and VIIIA are of interest, particularly with metals such as cobalt, nickel, vanadium, tungsten, titanium and molybdenum, for example, platinum-tungsten, platinum-nickel or platinum-nickel-tungsten.

The foregoing metals may be incorporated into the catalyst by any suitable method such as impregnation or exchange onto the zeolite. The metal may be incorporated in the form of a cationic, anionic or neutral complex such as $Pt(NH_3)_4{}^{2+}$ and cationic complexes of this type will be found convenient for exchanging metals onto the zeolite. Anionic complexes such as the vanadate or metatungstate ions are useful for impregnating these metals into the zeolites.

The amount of the hydrogenation-dehydrogenation component is suitably from 0.01 to 10 percent by weight, normally 0.1 to 5 percent by weight, although this will, of course, vary with the nature of the component, less of the highly active noble metals, particularly platinum, being required than of the less active base metals.

In practicing the process of the invention, it may be desirable to formulate each component of the catalyst system with another material resistant to the temperature and other conditions of the process. Such matrix materials include synthetic or naturally occurring substances as well as inorganic materials such as clay, silica and/or metal oxides. The latter may be either naturally occurring or in the form of gelatinous precipitates or gels including mixtures of silica and metal oxides. Naturally occurring clays, which can be composited with the zeolite include those of the montmorillonite and kaolin families, which families includes the sub-bentonites and the kaolins commonly known as Dixie, McNamee : Georgia and Florida clays or others in which the main mineral constituent is halloysite, kaolinite, dickite, nacrite or anauxite. Such clays can be used in a raw state as originally mined or initially subjected to calcination acid treatment or chemical modification.

In addition to the foregoing materials, the zeolites employed herein may be composited with a porous matrix material, such as alumina, silica-alumina, silica-magnesia, silicazirconia, silica-thoria, silica-berylia, silica-titania as well as ternary compositions, such as silica-alumina-thoria, silicaalumina-zirconia, silica-alumina-magnesia and silica-magnesiazirconia. The matrix may be in the form of a cogel. The relative proportions of zeolite component and inorganic oxide gel matrix may vary widely with the zeolite content ranging from between 1 to 99 percent by weight and more usually in the range of 5 to 80 percent by weight of the composite.

Xylene isomerization feeds, processed in accordance with the invention, are any aromatic $C_8$ mixture containing ethylbenzene and xylene(s). Generally, such a mixture will have an ethylbenzene content in the range of 0 to 50 weight percent, an ortho-xylene content in the range of 0 to 35 weight percent, a meta-xylene content in the range of 20 to 95 weight percent, and a para-xylene content in the range of 0 to 15 weight percent. The feed in addition to the above aromatic $C_8$ mixture can contain non-aromatic hydrocarbons, such as paraffins and naphthenes. The paraffins will comprise 0 to 20 weight percent of the feed ; generally, the paraffins will comprise $C_8$-$C_9$ paraffins.

In accordance with the invention such a feed is contacted with a catalyst system under conversion conditions including a temperature of 200 to 600°C (400-1100°F), a pressure of 100 to 10450 kPa (0-1500 psig), a WHSV of 0.5 to 100 ; and a $H_2$/HC (feed) molar ratio of 0 to 10. Preferably, those conditions include a temperature of 260 to 510°C (500 to 950°F), a pressure of 270 to 2860 kPa (25 to 400 psig), a WHSV of 3 to 50 and $H_2$/HC molar ratio of 0.5 to 5. The conversion process may be carried out as a batch type, semi-continuous or continuous operation.

In general, the xylene isomerization reaction is carried out in a fixed bed flow reactor containing the catalyst system described above. The two component catalyst system may be used as a mixture. Such a mixture can be a physical mixture of two preformed zeolites ; alternatively, the zeolite mixture can result as the product of cocrystallization of a zeolite synthesis reaction mixture. In another embodiment the two components of the catalyst system are in sequential beds. That is, the component (A) of the catalyst system most selective for converting ethylbenzene forms one part of the bed, while the other compoent (B) of the catalyst system which is most effective to isomerize the xylene components forms the remaining portion of the catalyst bed. Thus, the conversion process of the invention could be carried out in two different reactors, that are either at the same or different temperatures. However, preferably, the feed is cascaded over the two catalysts disposed sequentially in a single reactor. In cascading, the feed is contacted with the two components of the catalyst system without intervening separation of light gases.

It is preferred that, the component (A) of the catalyst system effective to deethylate and disproportionate ethylbenzene is upstream with respect to the catalyst component (B) which is effective to isomerize the xylene components of the $C_8$ aromatic feed.

After the conversion process the isomerization product can be treated to isolate para-xylene. Thus, the isomerizate product can be fed to a crystallizer to crystallize para-xylene and thus the para-xylene can be isolated. The residual isomerizate can then be stripped of products lighter than $C_8$. Products heavier than $C_8$ in the residual isomerizate can be further processed. $C_8$ fractions from which para-xylene has been removed can be recycled to the isomerizer.

One result of the process of the invention is to convert the mixed xylene components of the feed containing p-xylene in an amount less than that at thermodynamic equilibrium to an extent such that product effluent from the isomerizer contains p-xylene in an amount at least approaching that of p-xylene in the xylene mixture produced at thermodynamic equilibrium. Moreover, xylene yields are at least comparable and most often greater than those of prior isomerization processes.

## EXAMPLES

The following examples illustrate xylene isomerization of the invention. The xylene isomerization was conducted at 1480 kPa (200 psig), $H_2$/HC = 2 and at 396°C except for the pure HZSM-5 which was tested at 360°C. The space velocity was varied to provide a range of conversion levels. From the resulting data, xylene loss [percent xylenes lost with respect to xylenes in the feed] and PE(p-xylene) [para xylene percent of thermodynamic equilibrium with respect to the xylenes in the product] values were obtained by interpolation. The feed consisted of 13.9% ethylbenzene, 10.4% p-xylene, 52.9% m-xylene and 22.6% o-xylene.

## Example 1

The xylene isomerization properties of a HZSM-22 catalyst ($SiO_2$/$Al_2O_3$ = 70, crystal size about 1.5 microns) was tested as described above. At 30% ethylbenzene conversion the xylene loss was 0.88% and PE(p-xylene) was 77%. This catalyst shows exceptionally low xylene loss but has low xylene isomerizaion activity as the p-xylene is 23% from equilibrium.

## Example 2

The xylene isomerization properties of a HZSM-5 (SiO$_2$/Al$_2$O$_3$ = 70, crystal size about 0.2 microns) catalyst was tested as described above. At 30% ethylbenzene conversion the xylene loss was 2.82% and PE(p-xylene) was 102%. This catalyst shows normal xylene loss and very good xylene isomerization activity.

## Example 3

The xylene isomerization properties of a catalyst comprising a physical mixture of 90 weight percent of the catalyst of Example 1 and 10 weight percent of the catalyst of Example 2 was tested as described above. At 30% ethylbenzene conversion the xylene loss was 1.26% and PE(p-xylene) was 95%. This catalyst has very low xylene loss and good xylene isomerization properties.

The combination of catalyst components A and B, as employed in Example 3 provides a synergistic combination of the best features of the individual components. This is also shown in Example 4 where a co-crystallized mixture of the two components, ZSM-5 and ZSM-22, is used and yields equilibrium xylenes with reduced xylene loss.

## Example 4

The xylene isomerization properties of a catalyst comprising a co-crystallized mixture containing 93 weight percent HZSM-22 and 7 weight percent HZSM-5 (for the composite, SiO$_2$/Al$_2$O$_3$ = 71, crystal size to about 0.05 microns) was tested as described above. At 30% ethylbenzene conversion the xylene loss was 2.05% and PE(p-xylene) was 100%. This catalyst shows low xylene loss and good xylene isomerization properties.

Example 5 illustrates the use of a test reaction to characterize and define the useful catalyst component A (e.g. ZSM-22).

## Example 5

Example 1 was repeated using several different space velocities to give various ethylbenzene conversions. At 10% ethylbenzene conversion, the para-xylene content of the xylene fraction in the effluent was 51% of its thermodynamic equilibrium value of 24%.

It is seen that under the test conditions specified in the application, the isomerization of para-xylene amounts to less than 80% of equilibrium at 10% ethylbenzene conversion. Under the same conditions, the ZSM-5 suitable for this invention as catalyst component B produces xylenes in an amount greater than 80% of equilibrium.

## Claims

1. A process for isomerizing a feed containing an aromatic C$_8$ mixture, which comprises xylene and from 0 to 50 percent ethylbenzene and in which the para-xylene concentration is less than that at thermodynamic equilibrium, which process comprises contacting the feed with a catalyst system comprising component (A) and component (B) wherein :

    component (A) comprises at least one zeolite and 0 to 10 weight percent of a hydrogenation metal, wherein said at least one zeolite produces less than 80 weight% equilibrium isomerization of para-xylene when tested under test conditions including a temperature of 370 to 480°C (700 to 900°F), a pressure of 100 to 4050 kPa (1 to 40 atmospheres), H$_2$ to hydrocarbon of 1 to 4, and a WHSV sufficient to produce 10 weight% ethylbenzene conversion with a feed of 14 weight% ethylbenzene, 10 weight% p-xylene, 53 weight% m-xylene and 23 weight% o-xylene ;

    component (B) comprises a further zeolite, different from the zeolite of component (A) and 0 to 10 weight percent of a hydrogenation metal, wherein said further zeolite produces greater than 80 weight% equilibrium isomerization of para-xylene when tested under said test conditions ; and

    the weight ratio of the zeolite of component (A) to the zeolite of component (B) ranges from 20/80 to 98/2 ;

    said contacting being undertaken at a temperature of 200 to 600°C (400 to 1100°F), a pressure of 100 to 10450 kPa (0 to 1500 psig), a WHSV of 0.5 to 100 and a H$_2$/HC molar ratio of 0 to 10.

2. The process of Claim 1, wherein the zeolite of each of components (A) and (B) has a Constraint Index of 1-12.

3. The process of Claim 1 or Claim 2, wherein the zeolite of component (A) is characterized by a xylene sorption capacity greater than 1 gram/100 grams of zeolite and an ortho-xylene sorption time for 30 percent of said capacity greater than 50 minutes, and the zeolite of component (B) is characterized by a xylene sorption capacity greater than 1 gram/100 grams of zeolite and an ortho-xylene sorption time for 30 percent of said capacity of less than 10 minutes, wherein the sorption capacity and sorption times are measured at 120°C and a xylene pressure of 4.5 ± 0.8 mm of mercury.

4. The process of any preceding Claim, wherein the zeolite of component (A) is ZSM-22, ZSM-23, ZSM-35 or ZSM-48 and the zeolite of component (B) is ZSM-5 or ZSM-11.

5. The process of any preceding Claim, wherein said feed is contacted with component (A) before it is contacted with component (B) of the catalyst system.

6. The process of Claim 5, wherein component (A) and component (B) are in separate reactors.

7. The process of Claim 5, wherein the component (A) and component (B) are in sequential beds in a fixed bed catalyst system.

8. The process of any preceding Claim, wherein said weight ratio is at least 50 : 50.

9. The process of any preceding Claim, wherein the $H_2/HC$ molar ratio is from 0.5 to 10.

10. The process of any preceding Claim, wherein the amount of metal in one or both of components (A) and (B) is from 0.01 to 2 weight percent.

## EUROPEAN SEARCH REPORT

Application Number

EP 90313794.1

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP - A1 - 0 234 684 (MOBIL OIL CORPORATION) * Claims 1-4,9,10; examples * | 1-10 | C 07 C 5/27 C 07 C 15/08 |
| A | EP - A1 - 0 057 607 (MOBIL OIL CORPORATION) * Claims 1-3,5,8,10,11,13; page 13, line 31 - page 14, line 17; examples * | 1,2,4, 9,10 | |
| A | EP - A1 - 0 109 962 (TORAY INDUSTRIES) * Claims 1,5-7,9-12 * | 1,4,9, 10 | |
| A | US - A - 4 695 667 (SUMITANI et al.) * Claims 1,3,4; examples * | 1,9,10 | |
| A | US - A - 4 700 012 (ONODERA et al.) * Claim 1; examples * | 1,9,10 | TECHNICAL FIELDS SEARCHED (Int. Cl.5) |
| A | EP - A1 - 0 151 351 (MOBIL OIL CORPORATION) * Claims 1,3,6,7; page 7, lines 17-22; examples * | 1,2,4, 9,10 | C 07 C 5/00 C 07 C 15/00 |
| A | EP - A1 - 0 102 716 (MOBIL OIL CORPORATION) * Claims 1-3,6-10 * | 1,2,4, 9,10 | |

The present search report has been drawn up for all claims

| Place of search | Date of completion of the search | Examiner |
|---|---|---|
| VIENNA | 20-03-1991 | PUSTERER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)